# EUROPÄISCHE PATENTSCHRIFT

(11) **EP 0 861 081 B1**
(45) Veröffentlichungstag und Bekanntmachung des Hinweises auf die Patenterteilung: **13.02.2002**
(21) Anmeldenummer: 96939018.6
(22) Anmeldetag: 13.11.1996
(51) Int. Cl.: A61K 31/55

(54) **VERWENDUNG VON EPINASTIN FÜR DIE BEHANDLUNG VON SCHMERZEN**
USE OF EPINASTINE IN THE TREATMENT OF PAIN
UTILISATION D'EPINASTINE POUR TRAITER LA DOULEUR

(30) Priorität: 14.11.1995 DE 19542281
(43) Veröffentlichungstag der Anmeldung: 02.09.1998
(73) Patentinhaber: Boehringer Ingelheim Pharma KG, 55218 Ingelheim am Rhein (DE); Boehringer Ingelheim International GmbH, 55218 Ingelheim am Rhein (DE)
(72) Erfinder: JUNG, Birgit, D-55270 Schwabenheim (DE); MEADE, Christopher, John, Montague, D-55411 Bingen (DE); PAIRET, Michel, D-88400 Biberach (DE)
(74) Vertreter: Laudien, Dieter, Dr.
(86) Internationale Anmeldenummer: EP9604957
(87) Internationale Veröffentlichungsnummer: WO9717971

(56) Entgegenhaltungen:
- EP-A- 0 035 749
- CLIN. EXP. PHARMACOL. PHYSIOL., Bd. 19, Nr. 1, 1992, Seiten 17-23, XP000650436 W. ROY JACKSON ET AL.: "Chemical design of peripherally acting compounds"
- PHARMACEUTICAL JOURNAL, Bd. 255, 21.Oktober 1995, Seiten 548-551, XP002024700 ALAN NATHAN: "A non-prescription medicines formulary 1: Analgesics"
- HUISARTS WET., Bd. 37, Nr. 4, 1994, Seiten 142-148, XP000650556 D.BIJL ET AL.: "Hoofdpijn, migraine en spanningshoofdpijn in de huisartspraktijk"
- DTSCH. APOTH.-ZTG, Bd. 125, Nr. 51-52, 1985, Seiten 2699-2707, XP002024701 D. SOYKA: "Migräne und Migränemittel"

## Beschreibung

Die Erfindung betrifft die Verwendung von Epinastin zur Herstellung eines Arzneimittels zur Behandlung und Prophylaxe von Schmerzen, insbesondere von chronischen bzw. durch Entzündungen bedingte Schmerzen sowie insbesondere von Migräne. Epinastin (3-Amino-9,13b-dihydro-1H-dibenz[c,f]imidazo[1,5-a]azepin Hydrochlorid) wurde von Fügner et al. [Arzneimittelforschung 38 (1988) 1446 - 1453] beschrieben. Dieser Wirkstoff kann in Form seines Racemats oder in Form der reinen Enantiomere oder als Gemisch aus verschiedenen Anteilen beider Enantiomere eingesetzt werden. Therapeutisch wird Epinastin als Hydrochlorid verwendet; die vorliegende Erfindung ist jedoch nicht auf das Hydrochlorid eingeschränkt, sondern betrifft jedes Säureadditionssalz mit einer pharmakologisch unbedenklichen Säure sowie die Verwendung der freien Base selbst.

Aus dem Stand der Technik ist die Verwendung von Epinastin und seiner Säureadditionssalze zur Behandlung des Asthmas bekannt. In der Europäischen Patentschrift EP-B-O 035 749 wird offenbart, daß dieser Wirkstoff außerdem für die therapeutische Behandlung von allergischen Erkrankungen wie allergischer Rhinitis, allergischer Konjunktivitis und allergischer Bronchitis geeignet ist.

Kopfschmerz ist ein häufig vorkommendes Symptom. In den meisten Fällen ist der Kopfschmerz von kurzer Dauer und mit schwachen Analgetika wie Acetylsalicylsäure, Paracetamol oder Ibuprofen gut beherrschbar. Dieser Kopfschmerz wird zwar als lästig empfunden, führt aber zu keiner wesentlichen Beeinträchtigung. Dagegen können chronisch wiederkehrende Kopfschmerzen wie Migräne zu solch starken Beeinträchtigungen führen, daß ein Arzt aufgesucht werden muß. Diese schwere Form des Kopfschmerzes läßt sich oft nicht angemessen mit einem schwachen Analgetikum behandeln.

Auf der anderen Seite gibt es kein allgemein akzeptiertes Klassifikationssystem für Kopfschmerzen; chronisch wiederkehrende Kopfschmerzen im Sinne der vorliegenden Erfindung beziehen sich vorwiegend auf Migräne und Bing-Horton Syndrom. Migräne selbst beinhaltet verschiedene Unterformen [J. Olesen und R.B. Lipton, Neurology 44 (1994) S6-S10 bezüglich einer Klassifikation]. Obwohl Migräne und Spannungskopfschmerzen zwei verschiedene Formen darstellen, werden sie von einigen Wissenschaftlern als ein klinisches Kontinuum angesehen mit Migräne an einem und Spannungskopfschmerz am anderen Ende des Spektrums [K.L. Kumar und T.G. Cooney : "Headaches" in "Medical Clinics of North America' Heft 79 Nr. 2 (1995) S. 261 bis 286]. Deshalb ist zu vermuten, daß viele Patienten mit Spannungskopfschmerz auch auf eine Migränetherapie ansprechen. Verschiedene andere Erkrankungen, die mit chronischen Schmerzen einhergehen, wie Neuralgien, Muskelschmerzen und Entzündungsschmerzen (wie zum Beispiel nach einem Sonnenbrand oder bei Arthrose bzw. auch nach Sportverletzungen) weisen mit dem chronisch wiederkehrenden Schmerz gemeinsame Mechanismen auf [Dray, A. Urban L. and Dickenson, A. Trends in Pharmacological Sciences 1994; 15:190-197].

Die gegenwärtige Therapie der Migräne beinhaltet den Einsatz von Secale-Alkaloiden (z.B. Ergotamin) und Sumatriptan. Obwohl viele Patienten von diesen Medikamenten profitieren, sprechen längst nicht alle Patienten darauf an. Außerdem treten zahlreiche Nebenwirkungen - wie Benommenheit und Schwindel - auf. Medikamente für die Migräneprophylaxe verkörpern Methysergid, Pizotifen, Betablocker (z. B. Propanolol) und KalziumkanalBlocker (z.B. Flunarizin). Chronische Anwendung von diesen Medikamenten geht mit Nebenwirkungen einher, die die Lebensqualität der Patienten beeinträchtigten; dabei können diese Medikamente im allgemeinen nur die Häufigkeit der Migräneanfälle vermindern, sie aber nicht völlig verhindern [H.C. Diener, Eur. Neurol. 34 (Supp 2) (1994) 18 - 25].

Es ist daher die Aufgabe der vorliegenden Erfindung, einen Wirkstoff für die Behandlung von Migräne bereitzustellen, der sowohl wirksam als auch frei von bedenklichen Nebenwirkungen ist. Eine weitere Aufgabe der vorliegenden Erfindung besteht darin, ein Medikament mit einem hohen Maß an Sicherheit für bestimmte Patientengruppen - wie Kinder und Patienten mit eingeschränkter Leber- und Nierenfunktion oder Herz-Kreislauferkrankungen- zur Verfügung zu stellen.

Überraschenderweise wurde nun gefunden, daß der Wirkstoff Epinastin diese Aufgaben in hervorragender Weise erfüllt. Dies wird durch folgende Versuchsergebnisse belegt:

In Versuchstieren kann eine Entzündungsreaktion in der Dura mater durch elektrische Reizung des Ganglion trigeminale ausgelöst werden, bedingt durch die Freisetzung von Neuropeptiden wie Substanz P aus den sensorischen Nervenendigungen. Die Plasmaextravasation kann mit Hilfe des Markers Evans Blau gemessen werden. Dieses Tiermodell wird im allgemeinen zum Auffinden von Substanzen, die für die Behandlung von Migräne geeignet sind, verwendet. Völlig überraschend war Epinastin in diesem Modell außerordentlich gut wirksam.

Ein häufig benutztes Tiermodell für entzündungsbedingte Schmerzen wurde erstmals von Randall und Selitto [L.O. Randall und J. J. Selitto, Arch. Int. Pharmacodyn 111, (1957) 409 - 419] beschrieben. Eine Entzündungsreaktion in der Rattenpfote wird induziert durch intraplantare Injektion von Hefezellen; anschließend wird die entzündungsbedingte Hyperalgesie gemesssen. Epinastin war auch in diesem Modell überraschenderweise gut wirksam.

Aus dem Stand der Technik ist Epinastin als Antihistaminikum bekannt. Die Interaktion zwischen einem Ligand (z.B. einem Wirkstoff) und einem Rezeptor kann mittels einer Affinitätskonstanten (Kᵢ) quantifiziert werden. Je niedriger der Wert der Affinitätskonstanten ist, desto fester ist die Bindung zwischen Wirkstoff und Rezeptor. Besondere Beachtung gilt denjenigen Bindungen, die einen Kᵢ Wert aufweisen, der niedriger oder in der gleichen Größenordnung der erwarteten Konzentration des Wirkstoffs im Zielgewebe (bzw. Plasma) ist. Der 5HT₇ Rezeptor ist ein besonderer Typ von 5-Hydroxytyptamin-bindenden Rezeptoren (für eine Klassifikation, siehe: 'Trends in Pharmacological Sciences 1994 Receptor & Ion Channel Nomenclature Supplement'). Überraschenderweise wird eine gute Bindung von Epinastin an den 5HT₇ Rezeptor gefunden. Die Kᵢ-Werte für Epinastin und zwei Vergleichsantihistaminika sind in Tabelle 1 aufgelistet.

Die beschriebene Erfindung wird nunmehr durch die folgenden Beispiele erläutert. Verschiedenartige, andere Ausgestaltungen werden für den Fachmann aus der vorliegenden Beschreibung ersichtlich. Es wird jedoch ausdrücklich darauf hingewiesen, daß die Beispiele und die Beschreibung lediglich zur Erläuterung vorgesehen und nicht als Einschränkung der Erfindung anzusehen sind.

### Beispiele:

### Untersuchung der Bindung von Epinastin an den 5HT₇-Rezeptor

Die Bindung von 2 nM ³H-LSD an einen Ratten-5HT₇-Rezeptor, der in CHO-Zellen exprimiert wurde, wurde für 60 min bei 37°C in 50 nM Tris-HCl Puffer, pH 7,4, der außerdem 10 mM MgCl₂, und 0,5 mM EDTA enthielt, gemessen. Die Reaktion wurde beendet mit rascher Vakuumfiltation durch Glasfaserfilter, die mit 0,1 % Polyethylendimmin vorbehandelt waren. Die Bindungsuntersuchungen wurden in Abwesenheit und in Anwesenheit von 6 bis 8 Konzentrationen von Epinastin, die zwischen 3 nM und 10 µM lagen, jeweils als Doppelbestimmungen durchgeführt. Für jede Konzentration wurde die Radioaktivität bestimmt, die sich auf dem Filter niederschlug, und mit dem Kontrollwert verglichen, um die Wechselwirkung von Substanz mit dem klonierten 5HT₇-Rezeptor zu bestimmen. Unspezifische Bindung wurde in Anwesenheit von 5-Carboxyamidotryptamin (5-CT) bestimmt. Die IC₅₀ für die Verdrängung des radioaktiven Liganden wurde durch graphische Extrapolation bestimmt und Kᵢ-Werte wurden berechnet nach einer Korrektur für die Verschiebung der Radioligandbesetzung nach der Formel von Cheng und Prusoff [Biochem. Pharmacol 22 (1973) 3099-3108]. Drei Experimente wurden durchgeführt. In jedem der Versuche zeigte Epinastin eine überraschend gute Bindung an den 5HT₇-Rezeptor. Für Epinastin betrug der mittlere Kᵢ-Wert 33 nM, für das (+)-Enantiomer 28 nM und für das (-)-Enantiomer 189 nM. Die individuellen Kᵢ-Werte für Epinastin und seine Enantiomere, die in den 3 Versuchen gefunden wurden, sind in Tabelle 1 zusammen mit den Kᵢ-Werten von zwei Vergleichsantihistaminika aufgelistet:

**Tabelle 1:**

| **Bindung von Epinastin (Racemat und Enantiomere) und zwei Vergleichsantihistaminika (Ketotifen und Mepyramin) an den 5HT**_{**7**} **Rezeptor** | | |
|---|---|---|
| Verbindung | Chemische Bezeichnung | Gemessene Kᵢ Werte, nM |
| Epinastin | 3-Amino-9,13b-dihydro-1H-dibenz-[c,f]-imidazo[1,5a]-azepin-Hydrochlorid (Racemat) | 27, 41, 30 |
| Epinastin (+) Enantiomer | (+)-3-Amino-9,13b-dihydro-1H-dibenz-[c,f]-imidazo[1,5a]-azepin-hydrochlorid | 45,18,22 |
| Epinastin (-) Enantiomer | (-)-3-Amino-9,13b-dihydro-1H-dibenz-[c,f]-imidazo[1,5a]-azepin-hydrochlorid | 155, 204, 207 |
| Ketotifen | | 406, 572, 331 |
| Mepyramin | | 2660, 747, 1330 |

### Untersuchung der Wirkung von Epinastin auf die durch elektrische Reizung des Ganglion trigeminale induzierte Plasmaextravasation in der Dura mater von Ratten

Männliche Wistar-Ratten mit einem Gewicht von 175- 190 g wurden mit Nembutal 50 mg/kg i.p. anästhesiert. Die V. jugularis wurde für die intravenöse Injektion von Substanzen kanüliert. Die Tiere wurden dann in einen stereotaktischen Rahmen plaziert. Symmetrische Bohrlöcher wurden gesetzt 3,0 mm lateral und 3,2 mm posterior vom Bregma, durch die die Elektroden 9,5 mm tief vorgeschoben wurden. 10 Minuten vor Beginn der elektrischen Reizung des rechten Ganglions trigeminale (5 min; 2 mA, 5 Hz, 5 ms) wurden die Testsubstanzen (Epinastin oder die Lösungsmittelkontrolle) intravenös verabreicht. Als Marker für die Plasmaproteinextravasation wurde 5 Minuten vor der elektrischen Reizung Evans Blau (30 mg/kg) ebenfalls intravenös verabreicht. 15 Min. nach Beendigung der elektrischen Stimulation wurde den Tieren 50 ml physiologische Kochsalzlösung über die linke Herzkammer perfundiert, um intravaskuläres Evans Blau auszuwaschen. Die Dura mater wurde entfernt, trocken getupft und gewogen. Evans Blau wurde aus dem Gewebe extrahiert mit 0,3 ml Formamid bei 50 ° C für 24 h. Der Farbstoffgehalt wurde photometrisch bei 620 nm Wellenlänge bestimmt, interpoliert mittels einer Standardkurve und dann als µg Evans Blau Gehalt pro mg Gewebe ausgedrückt.

### Datenauswertung:

Extravasation wurde ausgedrückt als Quotient aus dem Evans Blau Gehalt der stimulierten Seite und dem Evans Blau Gehalt der nichtstimulierten Seite. Die Ergebnisse sind als Mittelwert angegeben. Die Ergebnisse sind in Tabelle 2 aufgelistet.

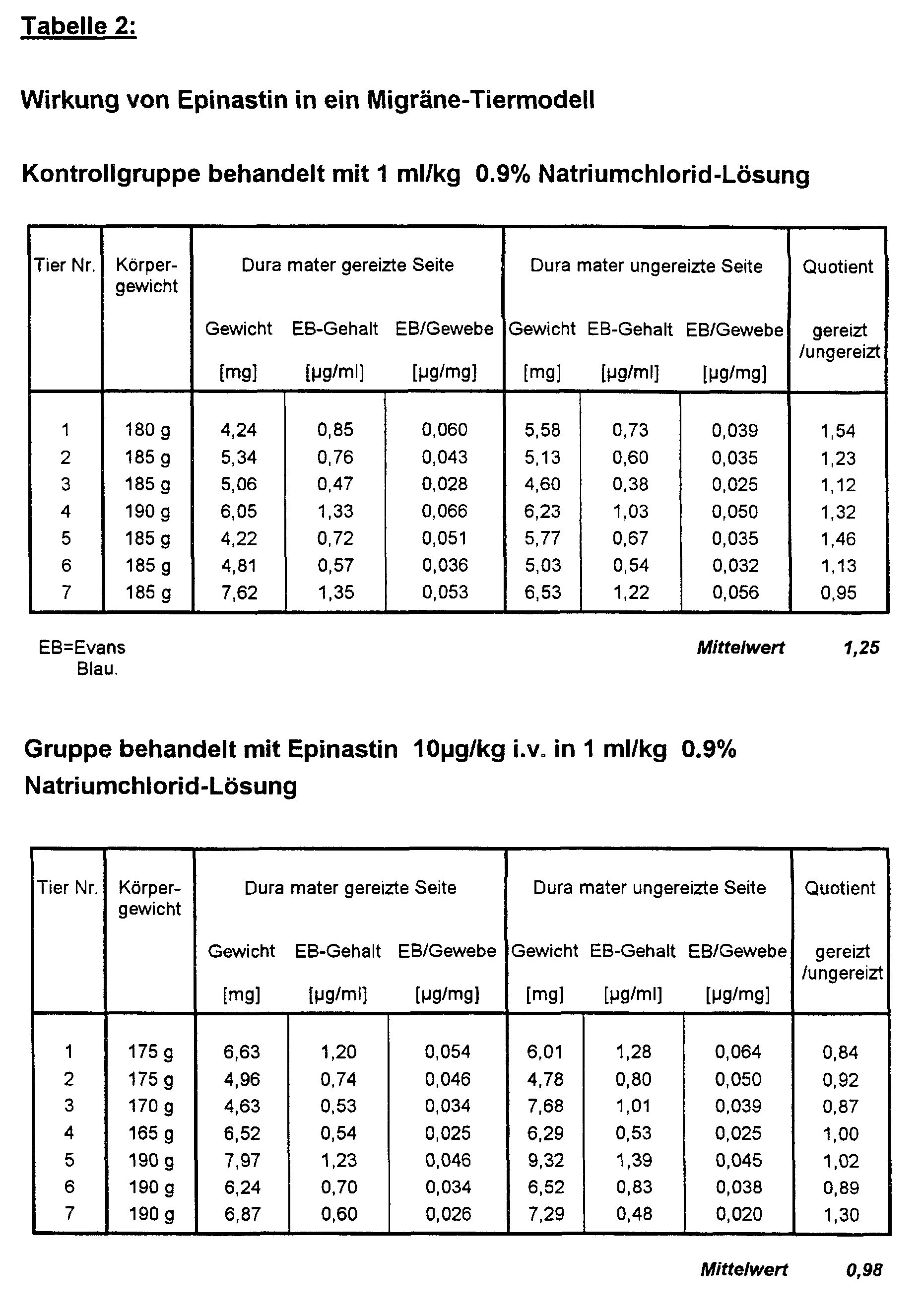

Die Tabelle 2 zeigt, daß in dem Tiermodell für Migräne die Behandlung mit Epinastin die durch elektrische Stimulation des Ganglion trigeminale induzierte Evans Blau Extravasation signifikant hemmte.

### Untersuchung von Epinastin auf die Hefe-induzierte Hyperalgesie in der Rattenpfote

Die Methode nach Randall und Selitto wurde modifiziert durch die Anwendung eines Analgesiemeters (Basile, Mailand).
Gruppen von 10 nüchternen Ratten des Stammes Chbb:THOM (Gewicht 110 - 140 g, 5 männlich, 5 weiblich) wurden oral 1 %iges Natrosol 250 HX (1 ml/100 g Körpergewicht) verabreicht, das 0, 0,3, 1,0, 3,0 oder 10 mg/kg Epinastin (Racemat) enthielt. Eine Stunde später erhielten die Tiere eine subplantare Injektion einer Hefezellsuspension in einem Volumen von 0,1 ml in die rechte Hinterpfote. 3 Stunden nach der Injektion der Hefesuspension wurde die Schmerzschwelle bestimmt, in dem ein Druck auf die entzündete Pfote so lange erhöht wurde, bis eine Schmerzäußerung erfolgte. Von der Schmerzschwelle, gemessen bei verschiedenen Dosen von Epinastin, wurde mittels einer Regressionsanalyse eine ED₅₀ ermittelt. Wie Tabelle 3 zeigt, erhöht Epinastin die Schmerzschwelle. Die Dosis Epinastin, die die Schmerzschwelle um 50% erhöhte, berechnete sich zu 1,1 mg/kg.

**Tabelle 3:**

| **Wirkung von Epinastin auf das Schmerzempfinden der entzündeten Rattenpfote** | | | | | |
|---|---|---|---|---|---|
| Substanz | Dosis mg/kg p.o. | Anzahl von Ratten | Mittelwert der Schmerzschwelle g / Hinterpfote | Standard Abweichung | % Zunahme |
| Kontrolle | 0,0 | 10 | 150,4 | 30,4 | |
| | | | | | |
| Epinastin | 0,3 | 10 | 164,8 | 41,8 | 9,6 |
| | | | | | |
| Epinastin | 1,0 | 10 | 235,2 | 51,5 | 56,4 |
| | | | | | |
| Epinastin | 3,0 | 10 | 259,4 | 60,7 | 72,5 |
| | | | | | |
| Epinastin | 10,0 | 10 | 272,8 | 60,9 | 81,4 |

**Tabelle 4:**

| **Test auf die analgetische Wirkung durch Randall Selitto** | | | | | | |
|---|---|---|---|---|---|---|
| Tierart: Ratte männlich / weiblich | | | | | | |
| Gewicht: 110 bis 140 g | | | | | | |
| Fütterung: nüchtern | | | | | | |
| Applikation: 1.0 ml/100 g p.o. | | | | | | |
| Vehikel: Natrosol 1% | | | | | | |
| 90 minütiger Wert | | | | | | |

| Substanz | Dosis mg/kg | N | Mittelwert | Wirkung in % | SD | VK in % |
|---|---|---|---|---|---|---|
| Kontrolle | 0/0 | 10 | 150 | | 46,188 | 30,792 |
| Epinastin / Ibuprofen | 0/3,0 | 10 | 153 | 2,00 | 20,028 | 13,090 |
| Epinastin / Ibuprofen | 0/10,0 | 10 | 202,00 | 34,67 | 29,740 | 14,723 |
| Epinastin / Ibuprofen | 0,3/0 | 10 | 216,00 | 44,00 | 44,020 | 20,380 |
| Epinastin / Ibuprofen | 0,3/3,0 | 10 | 252,00 | 68,00 | 57,116 | 22,665 |
| Epinastin / Ibuprofen | 0,3/10,0 | 10 | 277,00 | 84,67 | 81,792 | 29,528 |
| Epinastin / Ibuprofen | 1,0/0 | 10 | 232,00 | 54,67 | 57,889 | 24,952 |
| Epinastin / Ibuprofen | 1,0/3,0 | 10 | 295,00 | 96,67 | 70,593 | 23,930 |
| Epinastin / Ibuprofen | 1,0/10,0 | 10 | 369,00 | 146,00 | 107,233 | 29,060 |

Epinastin oder seine Enantiomere können für die Behandlung von Schmerzen als wässerige Injektionslösung (z.B. für intravenöse, intramuskuläre oder subkutane Applikation), als Tablette, als Suppositorium, als Salbe, als Pflaster für transdermale Applikation, als Aerosol für inhalative Anwendung über die Lunge oder als Nasenspray eingesetzt werden.

Der Wirkstoffgehalt einer Tablette oder eines Suppositoriums liegt zwischen 5 und 200 mg, vorzugsweise zwischen 10 und 50 mg. Bei Inhalation liegt die Einzeldosis zwischen 0,05 und 20 mg, vorzugsweise zwischen 0,2 und 5 mg. Bei einer parenteralen Injektion liegt die Einzeldosis zwischen 0,1 und 50 mg, vorzugsweise zwischen 0,5 und 20 mg. Die genannten Dosen können, falls erforderlich, mehrmals täglich gegeben werden. Besonders vorteilhaft könnte die Kombination mit anderen Therapieprinzipien sein, z.B. mit Acetylsalicylsäure oder Paracetamol oder NSAIDs wie Ibuprofen, Meloxicam, Indomethacin oder Naproxen; 5HT_{1D} Agonisten wie Sumatriptan, MK-462, Naratriptan oder 311C; CP-122,288; UK 116,044; Dopamin D₂ Rezeptorantagonisten wie z.B. Metoclopramid; Secale-Alkaloide wie Ergotamin, Dihydroergotamin oder Metergolin; Clonidin; Methysergid; Dotarizin; Lisurid; Pizotifen; Valproat, Aminotryptilin, Betablocker (z.B. Propranolol, Metoprolol); Kalziumkanalblocker (z.B. Flunarizin oder Lomerizin) oder Neurokinin-Antagonisten.

Im folgenden sind einige Beispiele für pharmazeutische Präparate mit dem Wirkstoff angegeben:
Tabletten:
   Epinastin 20 mg
   Magnesiumstearat 1 mg
   Mais Stärke 62 mg
   Laktose 83 mg
   Polyvinylpyrolidon 1,6 mg
Lösung zur Iniektion
   Epinastin 0,3 g
   Natriumchlorid 0,9g
   Aqua injectibilia ad 100ml
   Die Lösung kann unter Verwendung von Standardverfahren sterilisiert werden.
Wäßrige Lösung zur nasale oder inhalative Applikation
   Epinastin 0,3 g
   Natriumchlorid 0,9 g
   Benzalkoniumchlorid 0,01 mg
   Aqua purificata ad 100 ml

Die oben ausgeführten Lösung ist geeignet für die nasale Applikation in einem Spray, oder in Kombination mit einem Gerät, das ein Aerosol mit einer Partikelgröße vorzugsweise zwischen 2 und 6 µM produziert, zur Anwendung über die Lunge.

### Kapseln für die Inhalation

Epinastin wird in mikronisierter Form (Partikelgröße im wesentlichen zwischen 2 und 6 µM), gegebenfalis unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg Epinastin und 0 bis 40 mg Laktose eingefüllt.

### Inhalationsaerosol

Epinastin 1 Teil
Sojalecithin 0,2 Teile
Treibgasmischung ad 100 Teile

Die Zubereitung wird vorzugsweise in Aerosolbehälter gefüllt mit Dosierventil abgefüllt, der einzelne Hub wird so bemessen, daß eine Dosis von 0,5 mg abgegeben wird. Für die anderen Dosierungen des angegeben Bereichs verwendet man zweckmäßig Zubereitungen mit höherem oder niedrigerem Wirkstoffanteil.

### Salbe

Zusammensetzung g/100 g Salbe
Epinastin 2
Rauchende Salzsäure 0,011
Natriumpyrosulfit 0,05
Gemisch aus gleichen Teilen Cetylalkohol und Stearylalkohol 20
Weiße Vaseline 5
Künstliches Bergamotteöl 0,075
Destilliertes Wasser ad 100

Die Bestandteile werden in üblicher Weise zu einer Salbe verarbeitet.

## Patentansprüche

1. Verwendung von Epinastin in Form seiner Enantiomere, deren Mischungen oder des Racemats zur Herstellung eines Arzneimittels zur Behandlung von Schmerzen.

2. Verwendung von Epinastin nach Anspruch 1 zur Herstellung eines Arzneimittels zur Behandlung von Migräne, Bing-Horton Syndrom, Spannungskopfschmerzen, Muskelschmerzen, Entzündungsschmerzen oder Neuralgien.

3. Verwendung von Epinastin als Racemat oder in Form seiner Enantiomere vorliegend als freie Base oder als Säureadditionsssalz mit einer pharmakologisch unbedenklichen Säure zur Herstellung eines Medikaments zur therapeutischen Behandlung von Schmerzen.

4. Verwendung von Epinastin nach Anspruch 3 als Racemat oder in Form seiner Enantiomere vorliegend als freie Base oder als Säureadditionsssalz mit einer pharmakologisch unbedenklichen Säure zur Herstellung eines Medikaments zur therapeutischen Behandlung von Migräne, Bing-Horton Syndrom, Spannungskopfschmerzen, Muskelschmerzen, Entzündungsschmerzen oder Neuralgien.

5. Verwendung von Epinastin in Form seines Racemats oder seiner Enantiomere nach Anspruch 1 oder 2 in Kombination mit einem weiteren Schmerzmittel.

6. Verwendung von Epinastin in Form seines Racemats oder seiner Enantiomere nach Anspruch 5, **dadurch gekennzeichnet, daß** das in Kombination eingesetzte Schmerzmittel ein NSAID, ein 5HT_{1D} Agonist, ein Dopamin-D₂-Rezeptorantagonist, ein Secale-Alkaloid, ein Betablocker, ein Kalziumkanalblocker oder ein Neurokinin-Antagonist ist.

7. Verwendung nach Anspruch 6, **dadurch gekennzeichnet, daß** das NSAID Ibuprofen, Meloxicam, Indomethacin oder Naproxen ist.

8. Verwendung nach Anspruch 6, **dadurch gekennzeichnet, daß** der 5HT_{1D} Agonist Sumatriptan, MK-462, Naratriptan oder 311C ist.

9. Verwendung nach Anspruch 6, **dadurch gekennzeichnet, daß** der Dopamin-D₂-Rezeptorantagonist Metoclopramid ist.

10. Verwendung nach Anspruch 6, **dadurch gekennzeichnet, daß** das Secale-Alkaloid Ergotamin, Dihydroergotamin oder Metergolin ist.

11. Verwendung nach Anspruch 6, **dadurch gekennzeichnet, daß** der Betablocker Propranolol oder Metoprolol ist.

12. Verwendung nach Anspruch 6, **dadurch gekennzeichnet, daß** der Kalziumkanalblocker Flunarizin oder Lomerizin ist.

13. Verwendung nach Anspruch 6, **dadurch gekennzeichnet, daß** das in Kombination einzusetzende Schmerzmittel Acetylsalicylsäure, Paracetamol, Clonidin, Methysergid, Dotarizin, Lisurid, Pizotifen, Valproat, Aminotryptilin
CP-122,288 oder UK 116,044 ist.

## Claims

1. Use of epinastine in the form of its enantiomers, mixtures thereof or the racemate, for the preparation of a pharmaceutical composition for the treatment of pain.

2. Use of epinastine according to claim 1, for the preparation of a pharmaceutical composition for the treatment of migraine, Bing-Horton syndrome, tension headaches, muscular pains, inflammatory pains or neuralgias.

3. Use of epinastine as a racemate or in the form of its enantiomers, primarily as a free base or as an acid addition salt with a pharmacologically acceptable acid for the preparation of a medicament for the therapeutic treatment of pain.

4. Use of epinastine according to claim 3 as a racemate or in the form of its enantiomers, primarily as a free base or as an acid addition salt with a pharmacologically acceptable acid for the preparation of a medicament for the therapeutic treatment of migraine, Bing-Horton syndrome, tension headaches, muscular pains, inflammatory pains or neuralgias.

5. Use of epinastine in the form of its racemate or its enantiomers according to claim 1 or 2 in combination with another analgesic.

6. Use of epinastine in the form of its racemate or its enantiomers according to claim 5, **characterised in that** the analgesic used in combination is an NSAID, a 5HT_{1D}-agonist, a dopamine D₂ receptor antagonist, an ergot alkaloid, a beta blocker, a calcium channel blocker or a neurokinin antagonist.

7. Use according to claim 6, **characterised in that** the NSAID is ibuprofen, meloxicam, indomethacin or naproxen.

8. Use according to claim 6, **characterised in that** the 5HT_{1D} agonist is sumatriptan, MK-462, naratriptan or 311C.

9. Use according to claim 6, **characterised in that** the dopamine D₂ receptor antagonist is metoclopramide.

10. Use according to claim 6, **characterised in that** the ergot alkaloid is ergotamine, dihydroergotamine or metergoline.

11. Use according to claim 6, **characterised in that** the beta-blocker is propanolol or metoprolol.

12. Use according to claim 6, **characterised in that** the calcium channel blocker is flunarizine or lomerizine.

13. Use according to claim 6, **characterised in that** the analgesic drug used in combination is acetylsalicylic acid, paracetamol, clonidine, methysergide, dotarizine, lisuride, pizotifen, valproate, aminotryptiline,
CP-122,288 or UK 116,044.

## Revendications

1. Utilisation de l'épinastine sous forme de ses énantiomères, de leurs mélanges ou du racémate pour la préparation d'un médicament pour le traitement de la douleur.

2. Utilisation de l'épinastine selon la revendication 1 pour la préparation d'un médicament pour le traitement des migraines, du syndrome de Bing-Horton, des céphalées par tension nerveuse, des douleurs musculaires, des douleurs inflammatoires ou des névralgies.

3. Utilisation de l'épinastine sous forme de racémate ou de ses énantiomères sous forme de base libre ou de sel d'addition d'acide avec un acide pharmacologiquement acceptable pour la préparation d'un médicament pour le traitement thérapeutique de la douleur.

4. Utilisation de l'épinastine selon la revendication 3 sous forme de racémate ou de ses énantiomères sous forme de base libre ou de sel d'addition d'acide avec un acide pharmacologiquement acceptable pour la préparation d'un médicament pour le traitement thérapeutique des migraines, du syndrome de Bing-Horton, des céphalées par tension nerveuse, des douleurs musculaires, des douleurs inflammatoires ou des névralgies.

5. Utilisation de l'épinastine sous forme de son racémate ou de ses énantiomères selon la revendication 1 ou 2 en combinaison avec un autre antalgique.

6. Utilisation de l'épinastine sous forme de son racémate ou de ses énantiomères selon la revendication 5 **caractérisée en ce que** l'antalgique utilisé en combinaison est un AINS, un agoniste de 5HT_{1D}, un antagoniste de récepteur de dopamine D₂, un alcaloïde de l'ergot de seigle, un agent β-bloquant, un inhibiteur calcique ou un antagoniste de neurokinine.

7. Utilisation selon la revendication 6 **caractérisée en ce que** le AINS est l'ibuprofène, le méloxicam, l'indométhacine ou le naproxène.

8. Utilisation selon la revendication 6 **caractérisée en ce que** l'agoniste de 5HT_{1D} est le sumatriptan, MK-462, le naratriptan ou 311C.

9. Utilisation selon la revendication 6 **caractérisée en ce que** l'antagoniste de récepteur de dopamine D₂ est le métoclopramide.

10. Utilisation selon la revendication 6 **caractérisée en ce que** l'alcaloïde de l'ergot de seigle est l'ergotamine, la dihydroergotamine ou la métergoline.

11. Utilisation selon la revendication 6 **caractérisée en ce que** l'agent β-bloquant est le propanolol ou le métoprolol.

12. Utilisation selon la revendication 6 **caractérisée en ce que** l'inhibiteur calcique est la flunarizine ou la lomérizine.

13. Utilisation selon la revendication 6 **caractérisée en ce que** l'antalgique à utiliser en combinaison est l'acide acétylsalicylique, le paracétamol, la clonidine, le méthysergide, la dotarizine, le lisuride, le pizotifène, le valproate, l'aminotryptiline, CP-122288 ou UK 116044.
